Europäisches Patentamt

European Patent Office

Office européen des brevets

(19)

(11) Publication number:

**0 391 416**
**A1**

## (12) EUROPEAN PATENT APPLICATION

(21) Application number: **90106542.5**

(22) Date of filing: **05.04.90**

(51) Int. Cl.⁵: **C07K 3/26, C07K 15/06,**
**A61K 39/42, A23J 1/20,**
**A23C 9/13**

(30) Priority: **06.04.89 JP 87821/89**
**02.11.89 JP 286617/89**

(43) Date of publication of application:
**10.10.90 Bulletin 90/41**

(84) Designated Contracting States:
**AT BE CH DE DK ES FR GB IT LI NL SE**

(71) Applicant: **Chugai Seiyaku Kabushiki Kaisha**
**5-1, 5-chome, Ukima Kita-ku**
**Tokyo(JP)**

Applicant: **Kyodo Milk Industry Corporation**
**Limited**
**17-2, Koami-cho Nihonbashi**
**Chuo-ku Tokyo(JP)**

(72) Inventor: **Taniguchi, Hiromi**
**2-11-4, Higashi**
**Okegawa-shi, Saitama-ken(JP)**
Inventor: **Goto, Masamitsu**
**152-25, Imaizumi**
**Ageo-shi, Saitama-ken(JP)**
Inventor: **Okamoto, Toshiyuki**
**2-1044-2-202, Amanuma-cho**
**Ohmiya-shi, Saitama-ken(JP)**
Inventor: **Sakauchi, Iwao**
**3-8-1, Onta-cho**
**Higashimurayama-shi, Tokyo(JP)**
Inventor: **Ando, Tsuyoshi**
**155-296, Gokorokujitsu**
**Matsudo-shi, Chiba-ken(JP)**
Inventor: **Kirihara, Osamu**
**2-7-17, Sakurazutsumi**
**Musashino-shi, Tokyo(JP)**
Inventor: **Ando, Kunio**
**1877, Shimosakunobe Takatsu-ku**
**Kawasaki-shi Kanagawa-ken(JP)**

(74) Representative: **Vossius & Partner**
**Siebertstrasse 4 P.O. Box 86 07 67**
**D-8000 München 86(DE)**

(54) **Process for preparing a therapeutic agent for rotavirus infection.**

(57) Skimmed colostrum or colostrum whey is treated with an ultrafiltration membrane having a fractionation molecular weight of $5 - 15 \times 10^4$ so as to separate intact physiologically active proteins from the bovine milk. The yield of immunoglobulins contained in the separated proteins is about twice as high as the value achieved by the prior art and their quality is superior. The intact physiologically active proteins obtained by this method contain all immunoglobulins that have high neutralization antibody titres against the seven serotypes of rotavirus. Hence, rotavirus infection can be effectively prevented or treated by administering these proteins to humans or animals in the form of either a pharmaceutical agent or a beverage or food that contain such proteins.

EP 0 391 416 A1

# PROCESS FOR PREPARING A THERAPEUTIC AGENT FOR ROTAVIRUS INFECTION

The present invention relates to a method of separating and purifying biological active proteins from bovine milk, as well as a method of formulating in dosage form intact (not denatured) biologically active proteins that have been separated and purified from bovine milk by that method. The present invention also relates to a method of preventing and treating rotavirus infection with said intact physiologically active proteins.

Rotavirus which belongs to the family Reovirus was first isolated in 1973 from a child suffering from acute diarrheal disease. Rotavirus is a double-stranded RNA virus found in various mammals ranging from mice to monkeys and in birds such as chicks. It consists of an 11-segmented RNA having a molecular weight of $0.2 - 2.2 \times 10^3$, with protein units consisting of 8 - 10 peptides having molecular weights in the range of $15 - 130 \times 10^3$. The particles of this virus vary in diameter from 65 to 75 nm, with 32 capsomeres being arranged to extend radially from the central core in a wheel like shape (rota in Latin) and hence came the name "rotavirus".

Human rotavirus is held to be a major causative virus of acute non-bacterial gastroenteritis (e.g. pseudocholera or white diarrhea) that is epidemic among infants in the winter season (from November to March in the next year). Also being called "infantile gastroenteritis virus", human rotavirus is considered today as the main cause of group diarrheal disease among school-children. Rotavirus infection is characterized by severe diarrhea and vomiting and subsequent dehydration but in Japan where the level of national nutrition has improved remarkably, the cases of death of infants from rotavirus infection are now very rare. On the other hand, rotavirus infection is one of the major causes of infantile death in developing countries where poor nutrition is common. Further, it has recently become clear that diarrheal disease that is epidemic among elderly people in the summer season is also caused by rotavirus infection. Since the elderly have a lower resistance to infections than infants, it is not infrequent for infections to be fatal. In other words, diarrheal disease caused by rotavirus infection is closely related to the individual immunological potency and rotavirus diarrhea is a disease in "infants whose immune system is still immature" or in "elderly people with impaired immunological system".

Bovine rotavirus not only infects neonates causing severe diarrhea but also leads to fatal complication infections. The antigenicity of bovine rotavirus is complex and many types being identified, rendering it very difficult to prevent rotavirus infection by acquired immunity. Hence, prophylaxis by vaccination is hardly attained today and there is no effective means of prevention and treatment that can deal with every type of rotavirus.

Diarrheal disease caused by infection with rotavirus occurs mostly in the winter season and is several epidemic only in neonates. Feces from infected bovine contain a large amount of rotavirus and the comparatively prolonged excretion period of the virus, combined with its strong infectivity, increases the chances of rotavirus infection spreading easily.

Once rotavirus infection occurs, it becomes persistent and recurring. In barns that have been exposed to a rotavirus, mother cows possess high levels of rotavirus antibodies and their calves therefore receive passive immunity by antibodies passed on in colostrum. However, the number of antibodies in bovine colostrum rapidly decrease and soon disappear, resulting in the increased rate of rotavirus infection three or four days after birth, such development of infection probably falls on the days when the antibodies in bovine milk disappear. The antibodies transported from colostrum into blood are no longer effective in providing defense against infections such as rotavirus infection that attack the mucosa on the intestinal tract.

Rotavirus infection develops in 12 hours after birth in the earliest cases and is characterized by sudden excretion of watery feces and subsequent dehydration and weakness, but the patient usually recovers in 6 - 8 hours. Morbidity due to rotavirus infection is high but mortality rates are low, ranging from 0 to 50%. However, complicated infection with coronavirus, E. coli, pneumococcus and other pathogenic microorganisms frequently occurs, increasing the severity of the disease and aggravating its probable course. The lesion of the disease is localized in the small intestine, causing a marked change in villus epithelial cells.

As a result of intensive studies conducted in order to develop an effective method of preventing and treating rotavirus infection, the present inventors found that their objective could be attained by oral administration of an intact biological active protein in cow milk. The present invention has been accomplished on the basis of this finding.

Human rotavirus and the rotavirus that causes gastroenteritis in bovines or horses have been found by a complement-fixation reaction to have a common antigen. Mammal and bird rotaviruses are classified into seven serotypes I - VII, among which serotypes I - IV are particularly causative for their ability to infect humans and cause diarrheal disease. Considerable difficulties were previously encountered in detecting viruses but the recent advances in clinical chemistry have made it possible to detect virus antigens quickly by examining fecal extracts. Sera from convalescents turn out to be positive in a complement-fixation reaction with bovine rotavirus used as an antigen. In other words, it is suggested that the surface epitopes of human and bovine rotaviruse antigens have a part in common.

A large amount of immunoglobulins are known to be secreted in mammalian milk, particularly in colostrum. As already mentioned, young animals whose immune system is immature are infected with rotavirus more frequently than adults, so it would be expected for mammalian milk to contain a neutralizing antibody which recognizes an binds with the rotavirus antigen. In fact, it has been established that bovine milk which is the most common mammalian milk contains high titres of antibodies which protect against rotaviruses of the same species. Therefore, the success of an attempt to prevent or treat human rotavirus infection by means of a rotavirus antibody contained in bovine milk depends on whether the antibodies in bovine milk have the ability to neutralize human rotavirus (serotypes I - IV) notwithstanding the difference in animal species.

Rotavirus is known to be capable of active multiplication in cell cultures. The simplest way of determining the antibody titre for neutralizing rotavirus is a plaque reduction method in which a monolayer culture of cell line MA-104 obtained from monkey kidneys is infected with rotavirus and the number of plaques that form after cultivation for a given period is counted.

The principles of this method consist of infecting a monolayer culture of MA-104 cells with a virus and cultivating them after plating with an agar medium incorporating a dye that is capable of differentiating between viable and dead cells. Upon cultivation for a given period, the initial single virus multiplies on cells monolayer and the growing viruses spread outward, successively killing the infected cells. The agar medium contains a dye that stains viable cells but which does not stain dead cells, so the zone of dead cells produced by the multiplication of viruses can be observed as a plaque that is visually discernible from the surrounding area. In principle, a single virus produces a single plaque in this method, so the virus neutralizing activity of anti-virus substances including immunoglobulins can be measured by the following method. Various strains of rotavirus are mixed with immunoglobulins in bovine colostrum in a neutral buffer solution and the mixture is left to stand for a given period. If the immunoglobulins contain sufficient amounts of antibodies that neutralize rotavirus, the latter will form an antigen-antibody complex that is precipitated and removed from the system. Therefore, no plaque will form when the infections particles are completely removed. In practice, a maximum dilution ratio of the anti-virus active substance that is capable of reducing a 60% reduction plaque is called a "neutralization antibody titre" and is used as an index of in vitro anti-virus activity. The present inventors separated and purified immunoglobulin concentrates from bovine colostrum and measured their rotavirus neutralization antibody titres by the plaque reduction method. The results were as shown in Table 1. In the experiment, colostrum samples collected from several tens to hundreds of dairy cows were pooled and immunoglobulin concentrates were separated and purified for assaying the antibodies to the various strains of rotavirus. Some variations occurred between lots but the purified immunoglobulin concentrates showed without exception high neutralization antibody titres to all of the virus serotypes tested. Hence, it is clear that the immunoglobulin concentrates collected from the pooled colostrum samples should contain large amounts of antibodies that are capable of in vitro neutralization of various types of rotavirus including human rotavirus. In addition, the concentrates also contain lactoferrin, lactoperoxidase and other physiologically active proteins that were inherently present in bovine milk. For the sake of convenience, these physiologically active proteins are also referred to as "immunoglobulin concentrates" herein.

Table 1

| Rotavirus Antibody Titres of Immunoglobulin Concentrates from Bovine Colostrum | | | |
|---|---|---|---|
| Rotavirus strain | Serotype | Origin | Neutralization antibody titre |
| KU | I | human | 1:6250 - 1:31250 |
| Lincoln | I | bovine | 1:6250 - 1:31250 |
| SA-15 | III | monkey | 1:6250 - 1:31250 |
| SH-11 | III | horse | 1:6250 - 1:31250 |
| Odelia | IV | human | 1:6250 - 1:31250 |
| NCDV | VI | bovine | 1:6250 - 1:31250 |

These immunoglobulin concentrates having high rotavirus antibody titres can be produced by the method of the present invention in a far more economical way than by conventional methods. Conventionally, immunoglobulin concentrates from colostrum have chiefly been produced by precipitation with ammonium sulfate in the following manner. First, colostrum is adjusted to a pH of 4.5 and a milk-curdling enzyme rennet is added to precipitate casein. The floating milkfat is skimmed off and precipitating casein is removed by centrifugation. Ammonium sulfate is added to the thus obtained colostrum whey until 45% saturation is achieved, whereupon a fine precipitate of immunoglobulins forms. By centrifuging the mixture, fractions containing the immunoglobulins flow into a liquid portion having a higher specific gravity. The heavier liquid portion is passed through an ultrafiltration membrane having a fractionation molecular weight of $2 \times 10^4$ to remove ammonium sulfate and milk-derived low-molecular weight substances.

This conventional method, however, has the following serious problems.

(1) Low yield -- Immunoglobulins are not completely precipitated with ammonium sulfate and a very large portion of them is lost to the mother liquor.

(2) Lengthy procedure -- It takes a long time to remove the residual ammonium sulfate in the final concentrate by passage through an ultrafiltration membrane.

(3) Pollution treatment of liquid wastes is a time-consuming and expensive process -- the liquid wastes from plants contain large amounts of ammonium sulfate and hence require much cost and time for treatment.

In addition to these problems associated with the separation and purification of immunoglobulin concentrates, the following considerations must also be taken into account:

(4) the purified concentrate should contain antibodies capable of neutralizing the seven rotavirus serotypes I -VII;

(5) the timing of antibody administration;

(6) the dose of antibody to be administration; and

(7) a suitable pharmaceutical procedure for formulating the antibody.

The technical problem underlying the present invention is to provide physiologically active proteins or immunoglobulin concentrates. The solution of the above technical problem is achieved by providing these physiologically active proteins utilizing the method characterized in the claims. The physiologically active proteins produced by the claimed method can then be utilized to prevent and treat rotavirus infections in mammals. Further, these physiologically active proteins so-produced are of a high quality and are obtained in increased yields utilizing shorter process times without the need for time-consuming and expensive treatment of liquid wastes as compared to conventional methods.

The novel method of the present invention for separating and purifying biologically active proteins is the same as the prior art method up to the step of obtaining colostrum whey but it is characterized by immediate treatment of the colostrum whey with an ultrafiltration membrane having a fractionation molecular weight of $5 - 15 \times 10^4$, preferably $10 \times 10^4$. The principle of this method lies in fractionating milk proteins in accordance with their molecular weight. IgG1 which is contained in the largest amount in colostrum has a molecular weight of ca. $14 \times 10^4$ as a monomer. Upon examination by electrophoresis, however, most of the immunoglobulins present in the colostrum were found to exist in the form of a dimer or a trimer the two or three molecules of which bound to other proteins in the colostrum. Therefore, if the colostrum whey is treated with an ultrafiltration membrane having a fractionation molecular weight of $5 - 15 \times 10^4$, not only the low-molecular weight substances in the whey but also the greater part of milk-derived proteins such as $\alpha$-lactoalbumin, $\beta$-lactoglobulin and bovine serum albumin is eliminated; on the other hand, the proportion of the total protein occupied by immunoglobulins is more than doubled with the loss to the mother liquor being only less than 1%.

The immunoglobulin concentrate separated and purified by the procedure described above is subsequently freeze-dried or spray-dried to yield either white flakes or a white to yellow-white powder having a very small bulk specific gravity. The protein content of the powder depends on the immunoglobulin content of the colostal whey used but it is at least 90%, with 50 - 85% of which being occupied by intact (not denatured) immunoglobulins.

Rotavirus is classified into seven serotypes, I -VII, and its epidemicity has regional dependency. Thus, an immunoglobulin having a high neutralization antibody titre against rotavirus of type I is not necessarily effective in protecting against type II rotavirus infection. It is also known that the antibodies harbored by immunoglobulins vary greatly from one individual to another. Therefore, in order to confer passive immunity to rotavirus by oral administration of immunoglobulins, it is desirable to prepare immunoglobulins having high neutralization antibody titres against all serotypes of rotavirus. This can be accomplished by extracting immunoglobulins from a mixture of colostrum samples collected from the largest possible number of cows, preferably at least 50 cows, in various regions.

Whichever method is used to dry the thus obtained immunoglobulin concentrate, the resulting powder is not so hydrophilic and does not readily dissolve in water. Thus, even if the powder is encapsulated or formulated as tablets, it will form rubbery lumps and is not dispersed at all in an artificial gastric juice. This experimental result suggests that an aqueous solution of milk-derived pure immunoglobulins is extremely viscous compared to an aqueous solution of immunoglobulins purified from blood and that a simple dosage form of the former solution will merely adhere as rubber-like lumps to the mucosa on digestive tracts without being effectively dispersed in digestive fluids. Therefore, in order to administer immunoglobulins to humans or animals perorally and to have them exhibit a desired anti-viral activity, it is necessary to disperse or dissolve the immunoglobulins in the digestive tract. A pharmaceutical procedure that meets this requirement and which hence is used desirably comprises mixing one part of a fine powder of immunoglobulin concentrate with at least one part of a hydrophilic compound such as a sugar or a sugar alcohol, spraying the mixture with a small amount of water, mixing the ingredients thoroughly, and granulating the mixed product. The resulting granules have a hydrophilic sugar coat on the surfaces of hydrophobic immunoglobulin particles and hence have markedly improved dispersibility and solubilizability. In other words, a bovine immunoglobulin concentrate can not only be separated from bovine milk but also dissolve readily in it. In addition, a fine immunoglobulin powder can be rendered very highly soluble in warm water merely by mixing 0.5 - 50% of the powder with powdered milk. The water solubility of the fine immunoglobulin concentrate can also be improved by mixing it with a neutral (pH = 7) electrolyte powder such as a phosphate buffer saline powder.

Formulating immunoglobulins in dosage form is also essential for the purpose of administering them to bovine neonates perorally. The dosage form in this case should be such that not only is its peroral intake favored by bovine neonates but that also the physiologically active proteins including immunoglobulins in colostrum will reach the intended site of their action in digestive tracts to exhibit their activity. Digestive tracts in mammalian animals are covered with a mucosa layer and physiologically active proteins, if they are merely administered in aqueous solution, will not necessarily reach the intended site of action, i.e., the mucosa on the intestinal tract. The present inventors discovered two dosage forms that meet this requirement. The first suitable dosage form is based on a powder of the immunoglobulin-rich whey protein in colostrum, which powder is mixed with the powders of various electrolyte solutions such as Ringer's solution and Tyrode's solution, the mixture being dissolved in water just prior to use. One reason for adopting this dosage form is that although immunoglobulins are only slightly soluble in water, they dissolve fairly easily in electrolyte solutions. The second reason is that since bovine neonates suffering from rotavirus diarrheal disease have lost much water and electrolytes, it is therapeutically effective to supply these components simultaneously with immunoglobulins.

If one needs a further enhancement in the efficacy of immunoglobulins as a therapeutic agent, the following method may be employed. The physiologically active proteins of interest are dissolved in 1 - 50 volumes of Tyrode's solution whose glucose content is increased to 5 - 8%, and an oil or fat is added in an amount ranging from 1 to 3 times the amount of immunoglobulins, and the mixture is emulsified in the presence of lecithin or a cow milk fat globule membrane which are used as an emulsifier. The thus prepared emulsion of immunoglobulins is 2 - 3 times as effective in preventing rotavirus infection in bovine neonates as a mere solution of immunoglobulins in electrolyte solution. Further, it is particularly notable that a substantial amount of immunoglobulins is taken up by the body through the absorption barrier of the intestinal tract in spite of the fact that they are macromolecules.

Hence, the above-described method of permitting immunoglobulins or other physiologically active proteins to be taken up perorally after they are emulsified together with fats or oils is worth noting as a technique by which immunoglobulins can be absorbed through the intestinal tract.

The present inventors also investigated the efficacy of the above-described immunoglobulin concentrate in treating and preventing rotavirus diarrheal disease in human infants, calves and foals. In the case of humans, a powder containing 65% immunoglobulins was administered as an admixture with dry milk for babies or beverage in a dose of 100 - 1,200 mg (in terms of immunoglobulin) once a day, and its effectiveness in treating and preventing rotavirus infection was examined. While details of this investigation will be given later in the working examples, statistically significant results were attained in the following respects as regards the therapeutic effect of immunoglobulins: (1) reduction in the duration of diarrhea; (2) reduction in the duration of rotavirus excretion into feces. As for the preventive efficacy, the occurrence of rotavirus infection was significantly suppressed in a group to which the bovine-colostrum derived immunoglobulin concentrate was continuously administered in a preventive manner in a daily dose of 0.5 - 1.5 g (purity of immunoglobulin: 60%) per patient. These results clearly show that the immunoglobulin concentrate purified from bovine colostrum is effective in treating and preventing human rotavirus infection.

In the case of bovine, more than 100 calves were administered 25 g of bovine-colostrum derived immunoglobulin (65% pure) dissolved in an electrolyte solution and its effectiveness in treating and preventing rotavirus infection was investigated. In this case, too, the bovine colostrum derived immunoglobulins displayed significant efficacy in both therapeutic and preventive aspects.

In another experiment on bovine, the present inventors fed bovine neonates with sufficient amounts of colostrum immediately after their birth and then a whey protein fraction of the colostrum containing 50 - 65% immunoglobulins was administered perorally once a day within 24 hours of birth. It was found that this was a particularly effective method and that the development of rotavirus diarrheal disease could be effectively suppressed with the attendant prevention of complications.

Foals infected with rotavirus were also administered 25 g of immunoglobulins (65% pure) perorally on consecutive days to investigate their therapeutic effect against rotavirus infection. In this case, too, the "duration of diarrhea" and the "duration of rotavirus excretion into feces" were significantly shortened compared to a control group.

Thus, it was apparent that the bovine colostrum derived immunoglobulins exhibited trans-species efficacy in the treatment and prevention of rotavirus infection in mammals including humans.

The present invention is described below more specifically with reference to working examples.

Examples

Example 1 : Separation and Purification by Means of Ultrafiltration Membrane

Five hundred kilograms of bovine colostrum up to 48 hours postpartum was heated at 40°C and adjusted to a pH of 4.6 by addition of lactic acid. Thereafter, in accordance with the usual procedure of cheese production, 0.01% of a milk-curdling enzyme rennet and 0.02% of $CaCl_2$ were added and the mixture was stirred for 1 hour to insure that all of the casein content would form a fine curd. The solution containing the casein curd was centrifuged to separate into ca. 150 kg of the curd and 350 kg of the whey. Sodium hydroxide was added to the whey to adjust its pH to 6 - 7 and the mixture was passed through a filter press to obtain the filtrate. The filtrate was diluted three fold by being passed through an ultrafiltration membrane having a fractionation molecular weight of $10 \times 10^4$ and this concentration procedure was repeated five times to obtain 80 kg of the concentrate. The concentrate was aseptically filtered and freeze-dried to obtain about 8 kg of an immunoglobulin concentrate in powder form (purity, 65%).

The rotavirus neutralization antibody titre of this powder of immunoglobulin separated and purified from bovine colostrum was measured by the following procedure. First, bovine rotavirus (strain NCDV) multiplied in MA-104 cells was diluted to 200 PFU/ml. The virus dilution was mixed with an equal volume of a 5-fold serially diluted solution of the immunoglobulin concentrate and virus neutralization was effected by standing at 37°C for 1 hour. The amount of the neutralized viruses was measured by observing the formation of plaques on a monolayer culture of MA-104 cells that had been cultivated in a 3.5 cm dia. petri dish at 37°C for 2 days. The dilution ratio of the sample required for the number of plaques to decrease by about 60% as compared to the number of control plaques was adopted as the neutralization antibody titre of the immunoglobulin concentrate tested.

Example 2 : Formulation in Dosage Form Using Saccharide

Glucose (12 kg) was hydrated by spraying with distilled water (420 ml). The wetted glucose was thoroughly mixed with 4 kg of a fine powder of bovine immunoglobulin concentrate (63% pure as immunoglobulin; neutralization antibody titre against bovine rotavirus strain NCDV: 1/6250) that had been obtained by treatment with a pulverizer. The mixed product was granulated by means of an extrusion granulator furnished with a net having open areas of a size 2 mm (16 - 18 mesh). The granules were then dried with a fluidized dryer at 70°C for 25 minutes. The thus prepared granules had a water content of 7.5%.

Example 3 : Formulation in Dosage Form Using Nursing Conditioned Powder Milk

A commercial nursing conditioned powder milk (90 kg) was mixed with 10 g of a bovine immunoglobulin concentrate (Ig content, 65%; neutralization antibody titre against bovine rotavirus strain NCDV, 1/6250) and the mixture was thoroughly mixed as it was lightly wetted by spraying with 3 ml of distilled water. The mixed product was then dried at a temperature not higher than 50°C, pulverized by a suitable degree, and packed into moisture-proof bags containing single doses of 10 - 20 g.

Example 4 : Formulation in Dosage From by Mixing with Commercial Orange Juice

A commercial powdered orange juice (400 g) was mixed with 100 g of a finely ground bovine colostrum derived immunoglobulin concentrate (Ig content, 62%; neutralization antibody titre against rotavirus strain NCDV, 1/31250) and the mixture was subsequently processed as in Example 2 to prepare an orange juice powder containing bovine colostrum derived immunoglobulins.

Example 5 : Formulation in Dosage Form by Lactic Acid Fermentation

Ten grams of bovine colostrum derived immunoglobulin concentrate (purity, 62%; neutralization antibody titre against rotavirus strain NCDV, 1/31250) was added to 1,000 ℓ of commercial cow milk. After addition of a suitable amount of a mixed culture (starter) consisting of Lactobacillus bulgaricus and Streptococcus thermophilus, the mixture was cultivated at 40°C for 8 - 10 hours. As the microorganism multiplied, the pH of the system decreased. When the culture solution solidified totally, it was shipped as an edible product. The bovine immunoglobulins showed no inhibitory action on the growth of the Lactobacillaceae and Streptococcae. Since the cultivation time was short, the microorganism did not secrete a sufficient amount of protease to hydrolyze and thereby inactivate the bovine immunoglobulins. Thus, the initially added bovine immunoglobulins remained intact in the yogurt product prepared by the method of the present invention. Yogurt containing immunoglobulins could also be prepared by cultivation at about 20°C with mesophilic lactic acid bacteria, such as Streptococcus lactis and Streptococcus cremoris, being used as starters.

Example 6 : Treatment of Human Rotavirus Infection

The therapeutic efficacy of bovine colostrum derived immunoglobulins was investigated with juvenile outpatients who were taken care of by a pediatrician within 24 hours after the development of diarrheal disease in the winter season.

There is presently no therapeutic agent that is effective against human rotavirus infection and all therapies that are used today are symptomatic. Hence, in the treatment of the patients, the use of all nosotropic agents except antibiotics was permitted except in the case of complications by serious bacterial infections. The use of antibiotics was prohibited since they could modulate the therapeutic efficacy of immunoglobulins. The patients were administered the granules prepared in Example 2 (purity of bovine colostrum derived immunoglobulins, 67%; neutralization antibody titre against rotavirus, 1/6250) on a single-dose-a-day basis as a solution in either one of lukewarm water, nursing conditioned powder milk, cows' milk and fruit juice. Every time the patients excreted feces, a sample thereof was checked for appearance, nature and the presence or absence of rotavirus antigens. A group not administered the immunoglobulins was used as a control group. The two groups were compared' with respect to the duration of diarrhea and the time required for the rotavirus to disappear. The results are shown in Table 2. Both durations of diarrheal disease and virus excretion were significantly shortened in the treated group as compared to the control group. It was therefore clear that the bovine colostrum immunoglobulins were effective in curing human rotavirus infection.

Table 2

| Therapeutic Effect of Bovine Colostrum Derived Immunoglobulins for Human Rotavirus Infection | | | | | | | | |
|---|---|---|---|---|---|---|---|---|
| | n | Days to the disappearance of diarrhea | | | | | | |
| | | 2 | 3 | 4 | 5 | 6 | 7 | Average |
| Immunoglobulin* | 54 | 3 | 15 | 23 | 10 | 3 | 0 | 3.91 ± 0.96** |
| Control group | 78 | 0 | 0 | 17 | 31 | 22 | 8 | 5.27 ± 0.92 |
| | n | Days to the disappearance of fecal rotavirus | | | | | | |
| | | 2 | 3 | 4 | 5 | 6 | 7 | Average |
| Immunoglobulin* | 31 | 3 | 8 | 15 | 4 | 1 | 0 | 3.74 ± 0.93** |
| Control group | 51 | 0 | 0 | 13 | 22 | 11 | 4 | 5.10 ± 0.90 |

\* $P < 0.01$ in $\chi^2$ test
\** $P < 0.01$ in unpaired t-test

Example 7 : Prevention of Human Rotavirus Infection

In the four-week period from February 1 to 28 in 1986, 53 infants under two years of age were randomly divided into two groups. One group consisting of 30 infants was administered 4 g of glucose daily and the other group consisting of 23 infants was daily administered perorally 4 g of the bovine colostrum derived immunoglobulins formulated in granules by the method of Example 2 (1 g as immunoglobulin concentrate; neutralization antibody titre against rotavirus strain NCDV, 1/31250). Four weeks later, the frequency of the development of diarrheal disease caused by rotavirus infection was compared between the two groups. The results were as shown in Table 3.

Table 3

| Suppression of Rotavirus Infection by Bovine Colostrum Derived Immunoglobulins | | | |
|---|---|---|---|
| | n | Developments of diarrhea | Frequency |
| Control group | 30 | 16 | 53.3% |
| Treated group | 23 | 3** | 13.0% |

\** $P < 0.01$ in $\chi^2$ test

Diagnosis for the development of diarrheal disease in the winter season was finalized by checking the excretion of rotavirus into stools sampled from the patients. As Table 3 shows, the development of rotavirus infection was significantly suppressed in the group given daily oral doses of the bovine colostrum derived immunoglobulin preparation as compared with the control group. It was therefore clear that the bovine colostrum derived immunoglobulin preparation of the present invention was effective in preventing diarrheal disease that would otherwise be caused by rotavirus.

Example 8 : Prevention of Bovine Rotavirus Infection

One hundred and forty-one holstein calves not more than one month after birth were randomly divided into two groups. One group of calves were administered 25 g of bovine colostrum derived immunoglobulins (purity 63%) on a one-dose-a-day basis as a solution in either an electrolyte or glucose solution. The control group was administered only an electrolyte or glucose solution. The results were shown in Table 4.

Table 4

| Preventive Effect of Bovine Immunoglobulins against Diarrheal Disease in Calves | | |
|---|---|---|
| | n | Developments of Diarrhea |
| Control group | 58 | 31 |
| Immunoglobulin | 63 | 11** |

** $P < 0.01$ in $\chi^2$ test

Example 9 : Treatment of Rotavirus Infection in Bovine

Within 24 hours after the development of diarrhea that was indicative of rotavirus infection, holstein calves not more than one month after birth were administered perorally 25 g of bovine colostrum separated immunoglobulins (purity, 64%) on a one-dose-a-day basis for a continuous week as a solution in either water or an electrolyte solution. The duration of diarrhea (i.e., the time required for the disease to disappear) was compared between the treated group and the control group which were not administered the immunoglobulins. Calves were found to be positive for the development of rotavirus infection when bovine rotavirus antigens were detected in their fecal samples. As Table 5 shows, the bovine colostrum derived immunoglobulins were capable of significantly shortening the duration of diarrheal disease caused in calves by rotavirus infection.

Table 5

| Therapeutic Effect of Bovine Immunoglobulins for Rotavirus Infection | | |
|---|---|---|
| | n | Duration of diarrhea (mean ± S.D.) |
| Immunoglobulin | 34 | 3.2 ± 0.16** - 37.3% |
| Control group | 39 | 5.1 ± 2.38 |

** $P < 0.01$ in Student t-test

Example 10 : Prevention of Rotavirus Infection in Horses

Nineteen Thoroughbred foals born in Hidaka, Hokkaido were randomly divided into two groups, one of which was administered perorally 25 g of bovine colostrum derived immunoglobulins on a one-dose-a-day basis for 10 continuous days starting on the first week of birth. The other group was a control. The two groups were checked for the development of diarrhea due to rotavirus infection.

Table 6

| Preventive Effect of Bovine Colostrum Derived Immunoglobulins against Diarrhea in Foals | | |
|---|---|---|
| | n | Developments of Diarrhea |
| Immunoglobulin | 10 | 2 |
| Control group | 9 | 6 |

The difference between the two groups was not statistically significant but the neutralization antibody titre of the bovine immunoglobulins and the results of the experiments on humans and calves clearly indicate that the bovine immunoglobulins were effective in preventing rotavirus infection in horse.

Example 11

One hundred and fifty kilograms each of the frozen colostrum specimens collected from southern Kyushu, Nagano Prefecture and the Tokachi plain in Hokkaido were mixed and heated at 40° C to form a solution, the pH of which was adjusted to 4 by addition of acetic acid. The mixture was then centrifuged to remove cream. In accordance with the usual practice of cheese production, 0.01% of rennet and 0.02% of $CaCl_2$ were added to the skimmed colostrum and the mixture was stirred for 1 hour to completely curdle the casein. The solution was further centrifuged to separate into the coagulated casein curd (130 kg) and a whey portion (300 kg). Sodium hydroxide (NaOH) was added to the whey to adjust its pH to 6 - 7. Thereafter, the whey was passed through a filter press to obtain a filtrate. The filtrate was passed through an ultrafiltration membrane having a fractionation molecular weight of $10 \times 10^4$ to concentrate the filtrate by three folds. This procedure was repeated five times to obtain 80 kg of a concentrate. The concentrate was filtered aseptically and freeze-dried to obtain a powder of colostrum whey protein in an amount of ca. 8 kg. This powder contained 95% protein, 65% immunoglobulins and 5.7% lactoferrin.

Table 7

| Rotavirus Neutralization Antibody Titres of Protein Powders Prepared from Colostrum Specimens in Various Regions | | | | |
|---|---|---|---|---|
| Rotavirus serotype | Anti-virus activity* | | | |
| | Southern Kyushu | Nagano | Tokachi | Mixture |
| I | 1:31250 | 1: 6250 | 1: 1250 | 1:31250 |
| II | 1: 1250 | 1: 1250 | 1: 6250 | 1: 6250 |
| III | 1:31250 | 1: 6250 | 1: 1250 | 1:31250 |
| IV | 1: 6250 | 1: 6250 | 1: 6250 | 1: 6250 |
| V | 1: 1250 | 1: 6250 | 1:31250 | 1:31250 |
| VI | 1: 6250 | 1:31250 | 1: 1250 | 1: 6250 |
| VII | 1: 6250 | 1: 6250 | 1:31250 | 1: 6250 |

* Anti-virus activity: The dilution ratio of 1 g of immunoglobulin that achieved 60% reduction in the number of plaques formed by infection of MA-104 cells with rotavirus

As Table 7 shows, the immunoglobulins in the colostrum specimens collected from dairy cows in southern, middle and northern regions of Japan had characteristic rotavirus neutralization antibody titres in that the immunoglobulins in the colostrum from southern Kyushu had high neutralization antibody titres against serotypes I and III whereas the immunoglobulins in the colostrum from Hokkaido had low neutralization antibody titres against these serotypes but exhibited high neutralization antibody titres against serotypes V and VII. In contrast, the immunoglobulins in the colostrum from Nagano Prefecture situated in the central part of Honshu (mainland Japan) showed intermediate neutralization antibody titres.

Hence, by mixing the colostrum specimens collected from these three regions of Japan, whey protein powders could be obtained that exhibited strong mean anti-virus activities against the seven serotypes of rotavirus as shown in Table 7.

The whey proteins extracted from mixtures of the colostrum specimens collected from the three regions mentioned above were administered perorally to Japanese native black cow in the respective regions in the winter season where rotavirus infection prevailed and their effectiveness in protecting against the infection was investigated. Within 24 hours of birth, the neonates were given perorally the first dose of immunoglobulins (5 g) dissolved in 300 ml of an electrolyte replenisher containing 6% glucose. Thereafter, the neonates were orally administered the immunoglobulins for 3 - 5 consecutive days on a one-dose-a-day basis. The control group was administered only 300 ml of an electrolyte replenisher containing 6% glucose. The calves were kept under observation for one month and any development of diarrhea and cases of death from infection were recorded.

Table 8

| Effectiveness of Colostrum Whey Proteins in Preventing the Development of Diarrhea and Death from Infection in Japanese Native Black Cow | | | | | | |
|---|---|---|---|---|---|---|
| | n | Severity of diarrhea | | | Cases of survival | Cases of death |
| | | - | + | + + | | |
| Immunoglobulin | 62 | 9 | 19 | 34 | 53 | 9 (14.5%) |
| Control group | 134 | 0 | 33 | 101 | 59 | 75 (56.0%) |
| Statistical difference | $P<0.01$ in $\chi^2$ test | | | | $P<0.01$ in $\chi^2$ test | |

As is clear from Table 8, the development of diarrhea and the cases of death from infection could be significantly reduced by administering colostrum whey proteins.

Example 12

Immunoglobulin-rich whey proteins in powder form were prepared from bovine colostrum by the separation and concentration procedures employed in Example 11. One part of these whey proteins was mixed with 6 parts of a glucose-enriched Tyrode's solution in powder form. The powdered glucose-enriched Tyrode's solution had the following composition: 5.045 g glucose; 0.800 g sodium chloride; 0.02 g potassium chloride; 0.01 g magnesium chloride; 0.02 g calcium chloride; 0.005 g monobasic sodium phosphate; and 0.100 g sodium bicarbonate. The completed powder mixture was lightly wetted by uniform spraying with distilled water in an amount corresponding to 3 - 4% of the total weight. The mixture was thoroughly kneaded and granulated on an extrusion granulator equipped with a net having open areas of a size of 2 mm (16 - 18 mesh). The granules were dried on a fluidized dryer at 70°C for 25 minutes. The thus prepared granules had a water content of 5.5 - 7.5%.

Example 13

Granules of whey proteins (37.5 g) that were rich in colostrum immunoglobulins were prepared as in Example 12. They were dissolved in 500 ml of water and administered orally to holstein bovine neonates that were not more than 24 hours of birth. Thereafter, 37.5 g of the whey protein granules in 500 ml of water was administered orally to the calves for 5 consecutive days on a one-dose-a-day basis. The control group was administered only with an electrolyte replenisher that was free from whey proteins. After the completion of the administration, the calves were observed for 4 weeks and any development of diarrhea and cases of death from infection were recorded.

Table 9

| Effectiveness of Colostrum Whey Proteins Preparations in Preventing the Development of Diarrhea and Death from Infection in Holstein Neonates | | | | | | | |
|---|---|---|---|---|---|---|---|
| | n | Severity of diarrhea | | | | Cases of survival | Cases of death |
| | | - | ± | + | + + | | |
| Whey protein granules | 37 | 25 | 6 | 3 | 3 | 37 | 0 |
| Control group | 45 | 9 | 12 | 11 | 13 | 43 | 2 |
| Statistical difference | P<0.01 in $\chi^2$ test | | | | | n.s. | |

Table 9 clearly shows that whey proteins extracted from bovine colostrum are effective in preventing rotavirus diarrhea.

Example 14

The anti-virus activity of the immunoglobulin-rich whey proteins that were extracted from the colostrum of dairy cows kept in Nagano Prefecture was enhanced by the following pharmaceutical procedure. First, 22.5 g of the colostrum whey protein prepared in Example 12 was dissolved in 300 ml of water. After addition of soybean oil (6 g) and milk fat globule membrane (0.3 g), the mixture was emulsified by sonication. The resulting emulsion (300 ml) was administered orally to holstein neonates within 24 hours after their birth. Starting on the next day, 300 ml of the emulsion was administered orally to the neonates for 5 consecutive days on a one-dose-a-day basis. The calves were subsequently observed for 4 weeks to check to see if rotavirus diarrhea would develop. Calves in the control group were orally administered an electrolyte replenisher alone. The colostrum whey proteins used analyzed as shown in Table 10.

Table 10

| Composition of Colostrum Whey Protein | |
|---|---|
| Ingredient | Content (%) |
| Proteins (total) | 75 - 95 |
| Immunoglobulins | 50 - 65 |
| lactoferrin | 3.5 - 6.0 |
| Lipid | <1.5 |
| Lactose | <3.0 |
| Ash | <1.0 |

Table 11

| Effect of Emulsified Whey Protein in Preventing Diarrhea | | | | | | | |
|---|---|---|---|---|---|---|---|
| | n | Severity of diarrhea | | | | Cases of survival | Cases of death |
| | | - | ± | + | + + | | |
| Whey protein | 12 | 11 | 1 | 0 | 0 | 12 | 0 |
| Control group | 15 | 1 | 2 | 6 | 6 | 15 | 0 |
| Statistical difference | P<0.01 in $\chi^2$ test | | | | | n.s. | |

As Table 11 shows, diarrhea due to rotavirus infection could be significantly prevented by oral administration of emulsified whey proteins. In Example 14, the daily dose of whey proteins was reduced from 5 g (Example 13) to 3 g but their preventive efficiency was comparable to or even better than in Example 13.

Example 15

The colostrum whey proteins prepared in Example 14 were converted to a powder form by the following procedure. First, 300 g of lactose and 100 g of whey protein isolate (WPI of Bioisolate, Inc.) were added to 5 ℓ of the emulsion prepared as in Example 13. The resulting solution was freeze-dried to form 800 g of a hygroscopic powder. When 16 g of this powder was added to 100 ml of water, followed by vigorous stirring, an emulsion containing 1 g of the colostrum whey protein could be readily reconstituted.

The present invention has many advantages over the prior art. As regards the separation and purification of physiologically active proteins from bovine milk, the present invention offers the following advantages:

(1) High yield -- No product loss occurs in the steps subsequent to the recovery of colostrum whey, so that the yield of immunoglobulins is about twice as high as in the prior art.

(2) Efficient effluent treatment -- The method of the present invention discharges very small amounts of liquid wastes as compared to the prior art, so that the effluent can be treated within a short time and at low cost.

(3) High quality -- The absence of the need for prolonged treatment enables the production of immunoglobulins that have better quality than those obtained by the prior art.

The present invention also provides a method of producing intact biologically active proteins that contain all immunoglobulins that have high neutralization antibody titres against the seven serotypes of rotavirus.

As regards the method of formulating in dosage form, the dispersibility and solubilizability of immunoglobulins can be markedly improved by coating their surface with a hydrophilic saccharide. The water solubility of immunoglobulin powder can be improved by mixing 0.5 - 50% of them either with a nursing conditioned powder milk or with a neutral (pH = 7) electrolyte powder. If the intact physiologically active protein is formulated in dosage form by mixing it in powder form with a powder of electrolyte solution, said active protein can be caused to efficiently reach the mucosa on the intestinal tract which is the intended site of its action. If desired, the intact physiologically active protein may be mixed with a neutral fat and emulsified with the aid of an emulsifier and the resulting emulsion will attain even higher effects than when the active protein is administered as a solution in electrolyte.

The intact physiologically active protein thus formulated in dosage form can be administered perorally in order to prevent or treat rotavirus infection in an effective way.

## Claims

1. A method of separating and purifying physiologically active proteins from milk, which method comprises treating skimmed colostrum or colostrum whey with an ultrafiltration membrane having a fractionation molecular weight of $5 - 15 \times 10^4$ so as to concentrate the physiologically active proteins in the colostrum which are chiefly composed of immunoglobulins.

2. The method of claim 1, wherein said milk is bovine milk.

3. The method of claims 1 or 2, wherein said colostrum or colostrum whey is prepared by mixing colostrum samples collected from milk obtained from different animal sources which are located in different geographic regions.

4. The method of any one of claims 1 - 3, wherein said ultrafiltration membrane has a fractionation molecular weight of $10 \times 10^4$.

5. Physiologically active proteins obtained by the method of any one of claims 1 - 4.

6. A composition comprising physiologically active proteins of claim 5 and a pharmaceutically acceptable carrier.

7. The composition of claim 6, wherein said carrier is a hydrophilic compound.

8. The composition of claim 7, wherein said hydrophilic compound is a saccharide.

9. The composition of claim 8, wherein said saccharide is glucose, maltose, sucrose, fructose or sorbitol.

10. The composition of any one of claims 6 - 9, wherein said composition is granulated.

11. The composition of claim 6, which is dry milk.

12. The composition of claim 11, which contains 0.5 - 50 of intact physiologically active proteins.

13. The composition of claim 6, which is a powder beverage.

14. The composition of claim 6, which is a yogurt-like food.

15. The composition of claim 6, wherein said physiologically active proteins are converted to a powder form.

16. The composition of claim 15, wherein said carrier is a powder of an electrolyte replenisher.

17. The composition of claim 16, wherein said electrolyte replenisher is Ringer's solution or Tyrode's solution.

18. The composition of claim 6, which is a milk substitute.

19. The composition of claim 6, which is an emulsion.

20. The composition of claim 19, wherein said emulsion is made comprising the steps of mixing one part said physiologically active proteins with at least one part of a fat and emulsifing said mixture in the presence of an emulsifier.

21. The composition of claim 20, wherein said fat is a neutral fat.

22. The composition of claim 21, wherein said neutral fat is butteroil or soybean oil.

23. The composition of any one of claims 20 - 22, wherein said emulsifier is lecithin or a milk fat globule membrane.

24. The composition of any one of claims 6 - 23 for the prevention and treatment of rotavirus infections, which contains an amount of said proteins sufficient to prevent and/or treat rotavirus infections.

25. The composition of claim 24, for the administration of 30 - 6,000 mg of said protein per day to a human.

26. The composition of claim 24 for the administration of 10 -500 mg/kg of said protein per day to a calf, piglet, puppy, kitten or any young mammal.

27. The composition of claim 24 for the peroral administration of 5 g of said protein to a calf at least once within 36 hours of birth and thereafter in a dose of at least 5 g daily for one week.

28. A method for the production of the composition of any one of claims 6 - 9, comprising the steps of adding a hydrophilic compound or a saccharide to fractions of said physiologically active proteins, wetting lightly the mixture by spraying with 1 - 5 of water, thoroughly mixing the wetted mixture, and granulating the mixed product.

29. A method for the production of the composition of claim 14, comprising the steps of adding said physiologically active proteins to bovine milk, inoculating the mixture with one or more Lactobacillaceae and/or Streptococcae microorganisms, and cultivating the mixture.

30. The method of claim 29, wherein the microorganisms are Lactobacillus, Streptococcus or Leuconostoc.

31. A method for the production of the composition of any one of claims 15 - 17, comprising mixing the powder form of the composition with a powder of an electrolyte replenisher.

32. The method of claim 31, wherein said electrolyte replenisher is Ringer's solution or Tyrode's solution.

33. The use of the physiologically active proteins of claim 5 for the preparation of the composition of any one of claims 6 - 27.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | WO-A-8 704 050 (LANIER INDUSTRIES, INC.) <br> * Claim 10; page 7, line 13 - page 8, line 30; page 18, lines 4-25; page 24, lines 4-9; page 26, lines 22-29; page 28, lines 6-14; page 46, line 21 - page 47, line 2; page 82, line 21 - page 83, line 26 * <br> --- | 1-33 | C 07 K 3/26 <br> C 07 K 15/06 <br> A 61 K 39/42 <br> A 23 J 1/20 <br> A 23 C 9/13 |
| A | EP-A-0 046 909 (SENDAI INSTITUTE OF MICROBIOLOGY) <br> * Claims 1,3,6-10; page 4, line 7 - page 5, line 7 * <br> --- | 1-33 | |
| A | FR-A-2 347 934 (BEHRINGWERKE AG) <br> * Claims 1-5; page 3, line 32 - page 4, line 27 * <br> ----- | 1-33 | |
| | | | TECHNICAL FIELDS SEARCHED (Int. Cl.5) <br><br> C 07 K <br> A 61 K |

The present search report has been drawn up for all claims

| Place of search | Date of completion of the search | Examiner |
|---|---|---|
| THE HAGUE | 13-07-1990 | RYCKEBOSCH A.O.A. |

EPO FORM 1503 03.82 (P0401)